# EUROPÄISCHE PATENTSCHRIFT

(11) **EP 2 926 676 B1**
(45) Veröffentlichungstag und Bekanntmachung des Hinweises auf die Patenterteilung: **08.04.2020**
(21) Anmeldenummer: 15165438.1
(22) Anmeldetag: 15.10.2004
(51) Int. Cl.: A41D 19/00, A61B 42/00, A61B 42/50

(54) **HANDSHUHPAAR**
PAIR OF GLOVES
PAIRE DE GANTS

(30) Priorität: 16.10.2003 AT 16312003
(43) Veröffentlichungstag der Anmeldung: 07.10.2015
(62) Teilanmeldung aus: 04789546.1
(73) Patentinhaber: Schrödl, Berthold, 1040 Wien (AT)
(72) Erfinder: Schrödl, Berthold, 1040 Wien (AT)
(74) Vertreter: Sonn & Partner Patentanwälte

(56) Entgegenhaltungen:
- AT-U- 6 608
- US-A- 4 876 747
- US-A- 5 467 483
- US-A- 5 579 539
- PATENT ABSTRACTS OF JAPAN Bd. 1996, Nr. 10, 31. Oktober 1996 (1996-10-31) -& JP 08, 158122, A, (MARUYAMA HAJIME), 18. Juni 1996 (1996-06-18)

## Beschreibung

Die Erfindung betrifft ein Handschuhpaar gemäß dem Oberbegriff des Anspruchs 1 sowie ein Verfahren zum Ausziehen eines solchen Handschuhpaars nach Anspruch 10.

In der US 4 876 747 A ist ein Handschuh beschrieben, der mit Hilfe eines Hakens ausgezogen werden kann, wobei die Handschuhe beider Hände bei Vorhandensein von zwei Haken auch gleichzeitig von den Händen des Benutzers abgezogen werden können. Dazu ist parallel zum Handschuhrand und auf der Handrückenseite der Stulpe eine Öffnung bzw. Schleife angebracht, in welche ein an der Wand oder dgl. montierter Haken eingeführt werden kann. Darüber hinaus kann diese Schleife aber auch von der anderen Hand ergriffen und der Handschuh abgezogen werden, zum Ausziehen des zweiten Handschuhs ist dann aber wieder der Haken erforderlich.

Ein Handschuh aus Elastomermaterial ist aus der US 5 579 539 bekannt geworden, der daumenseitig am Stulpenteil oder am dem Handrückenteil gegenüberliegenden Teil der Stulpe, allenfalls auch am Handrückenteil selbst, jeweils vom hinteren Rand der Stulpe beabstandet, eine Ausziehhilfe aufweist. Damit ist bei entsprechender ergonomisch und anatomisch ungünstiger Verdrehung der Hände und/oder Unterarme ein mühsames gleichzeitiges Abstreifen beider Handschuhe möglich. Durch die notwendige Verdrehung der Hände ist aber die Gefahr des Abrutschens von der Ausziehhilfe sehr gross, wobei dann die verschmutzte Außenseite des Handschuhs auf die Haut des Trägers zurückschnellen kann.

Ebenfalls eine Ausziehhilfe, hier in Form von bei normaler Benutzung abgedeckten, am Handrückenteil der Stulpe angebrachten Streifen, Fäden, Laschen od. dgl., ist in der US 5 467 483 beschrieben. Mit dieser Ausziehhilfe ist jedoch ein gleichzeitiges Abstreifen beider Handschuhe kaum möglich. Da jedoch nicht mit ausreichender Sicherheit gewährleistet werden kann, dass die Ausziehhilfe nicht doch nach Öffnen der Abdeckung beschmutzt wird, ist ein Ergreifen der Ausziehhilfe mit einer nicht mehr geschützten Hand mit dem Risiko der Beschmutzung dieser Hand verbunden.

Die JP 8-158122 zeigt einen Handschuh mit einer taschenförmig ausgebildeten Greifhilfe an der Handinnenseite, welche durch die Finger der anderen Hand ergriffen werden kann, wodurch der Ausziehvorgang erleichtert werden kann. Ein gleichzeitiges Abstreifen beider Handschuhe ist in diesem Dokument nicht geoffenbart und bei einer derartigen Anordnung einer Greifhilfe auch nicht einfach bzw. in keiner ergonomischen und einfachen Weise möglich. Beim Ausziehen der Handschuhe hintereinander kommt es unweigerlich beim Ausziehen des zweiten Handschuhs zur einer möglichen Beschmutzung der Finger der unbedeckten Hand.

Es war nun die Aufgabe der vorliegenden Erfindung, einen Handschuh der eingangs angegebenen Art derart zu verbessern, dass in ergonomischer und einfacher Weise ein Ausziehen der Handschuhe ohne Hilfsmittel auch bei sehr kurzen Stulpen ermöglicht wird, ohne dass die verschmutzte oder kontaminierte Außenseite mit der Haut des Trägers in Kontakt kommt. Auch das Anziehen der Handschuhe soll gleichzeitig erleichtert und vereinfacht werden, ohne dass der Träger die Außenseite beispielsweise steriler Handschuhe mit nicht sterilen Körperteilen oder Hilfsmitteln berühren muss.

Eine Lösung dieser Aufgabe kann durch die Merkmale erzielt werden, dass die Greifhilfe des anderen Handschuhs des Handschuhpaars an der Handrückenseite angeordnet ist, sodass die Greifhilfe jedes Handschuhs in der Stellung der gegengleich parallel gehaltenen Arme und Hände von der jeweils anderen Hand ergriffen und gleichzeitig und gegengleich ausgezogen und gleichzeitig automatisch gewendet werden, ohne dass die verschmutzte oder kontaminierte Außenseite der Handschuhe mit der Haut des Trägers in Kontakt kommt, und dass die Handschuhe des Handschuhpaars durch das weitere Ineinanderziehen miteinander verbunden werden. Dieser Winkelabstand betrifft das geometrische Zentrum der Greifhilfe, wohingegen der greifhilfefreie Abstand zwischen zwei Greifhilfen allenfalls einen kleineren Winkelbereich einschließen kann, insbesonders bei sehr ausgedehnten Greifhilfen. Durch diese geometrische Anordnung der Greifhilfe kann die Greifhilfe jedes Handschuhs in einfacher Weise in der natürlichen Stellung der gegengleich parallel gehaltenen Arme und Hände von der jeweils anderen Hand ergriffen und zum Abziehen des Handschuhs genutzt werden. Die allenfalls verschmutzte oder kontaminierte Außenseite der Handschuhe hat dabei keinen Hautkontakt, sondern wird nur mit der durch den anderen Handschuh geschützten Hand berührt. Die Handschuhe werden beim vorzugsweise gleichzeitigen und gegengleichen Ausziehen gleichzeitig automatisch gewendet, was die nachfolgende Handhabung vereinfacht und sicherer macht. Die Werte für den Winkelabstand der Greifhilfe gestatten für den überwiegenden Anteil von Handschuhträgern den ergonomisch günstigsten und auch am besten gegen unbeabsichtigtes Beschmutzen der Haut gesicherten Ausziehvorgang. Die Greifhilfe jedes Handschuhs ist als zur Innenseite des Handschuhs offene, hohle Ausbuchtung des Handschuhs ausgebildet. Auf diese Weise bildet sich beim auf der Hand befindlichen Handschuh eine Aufwölbung von dessen Material oder auch eine Falte, die dann mit der anderen Hand leicht ergriffen werden kann, im Gegensatz zu glatt anliegendem Material. Diese Greifhilfe kann im wesentlichen kreissymmetrisch oder auch länglich und allenfalls mit einer Vorzugsrichtung ausgebildet sein.

Die Greifhilfen auf beiden Handschuhen weisen einen Winkelabstand von zumindest 135° auf, vorzugsweise sind die Greifhilfen auf beiden Handschuhen einander im wesentlichen diametral gegenüberliegend angeordnet sind.

Gemäß einer weiteren Ausführungsform des erfindungsgemäßen Handschuhpaars erstreckt sich zum leichteren und sichereren Ergreifen die Greifhilfe jedes Hanschuhs über einen Winkelbereich von zumindest 90°.

Dabei könnte auch vorgesehen sein, dass die Greifhilfe jedes Handschuhs ein- oder mehrmals unterbrochen ist, wodurch sich in den meisten Fällen das Ergreifen und Halten der Greifhilfe verbessern lässt.

Vorteilhafterweise kann die Greifhilfe jedes Handschuhs mit die Griffigkeit erhöhenden Oberflächenstrukturen versehen sein. Dies kann durch entsprechendes Aufrauhen des Materials der Stulpen-Oberfläche geschehen als auch durch verschiedene Oberflächenstrukturen wie etwa Noppen, Rippen od. dgl. Auch sind viele unterschiedliche Formen dieser Oberflächenstrukturen möglich, etwa halbkugelförmige, im wesentlichen kegelförmige oder auch pyramidenförmige Noppen.

Sowohl bei zentralsymmetrischen als auch bei länglichen Greifhilfen kann das Festhalten dadurch erleichtert werden, dass die Greifhilfe jedes Handschuhs einen von der Oberfläche der Stulpe zumindest teilweise größer werdenden Querschnitt aufweist. Die Greifhilfe ist bei derartiger Ausbildung beispielsweise im Querschnitt ähnlich dem griechischen Buchstaben Omega oder ähnelt einem auf dem Kopf stehenden Kegel oder im Querschnitt einem auf der Spitze stehenden Dreieck.

Wenn gemäß einer vorteilhaften Ausführungsform der Erfindung im Bereich der Greifhilfe ein Dehnungsbereich der Stulpe vorgesehen ist, wird das erste Stadium des Abziehens des Handschuhs wesentlich erleichtert.

Bei separat gefertigten und anschließend mit der Stulpe des Handschuhs verbundenen Greifhilfen besteht auch zur Verbesserung der Griffigkeit der Greifhilfe die Möglichkeit, dass die Greifhilfe aus anderem, vorzugsweise griffigerem Material als die Stulpe des Handschuhs angefertigt ist.

Die Festigkeit des Handschuhs und damit die Sicherheit gegen Beschädigung und unbeabsichtigtes Beschmutzen der Haut des Trägers durch zerstörte Bereiche des Handschuhs hindurch kann verbessert werden, indem die Greifhilfe und/oder zumindest der Übergangsbereich zwischen Stulpe und Greifhilfe verstärkt ist, beispielsweise mittels eingelagerter Verstärkungsfasern.

In der nachfolgenden Beschreibung soll die Erfindung anhand der beigefügten Zeichnungen von Ausführungsbeispielen erfindungsgemäßer Handschuhe bzw. Handschuhpaare näher erläutert werden.

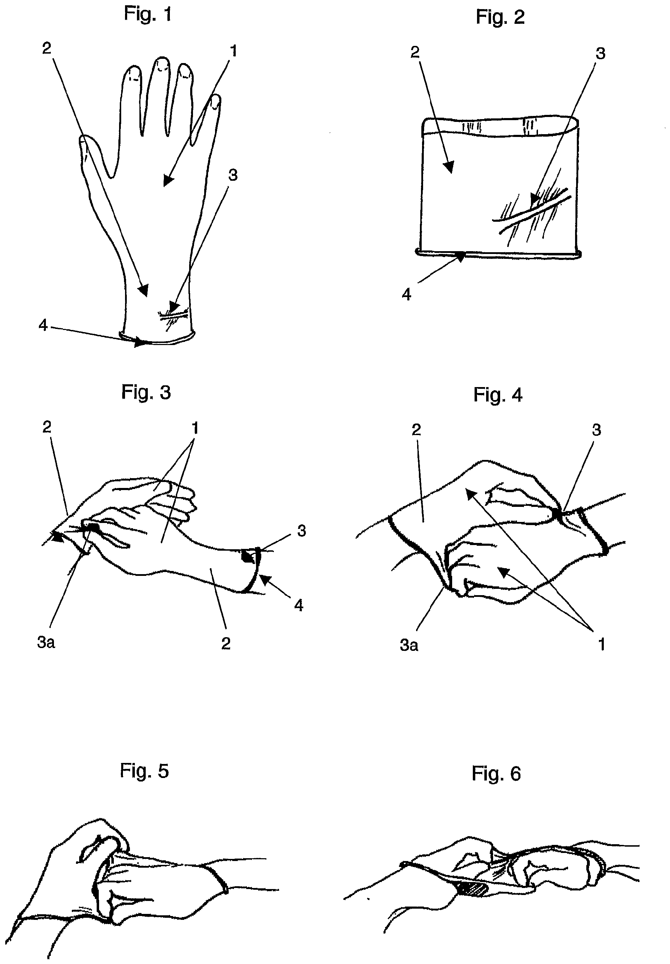

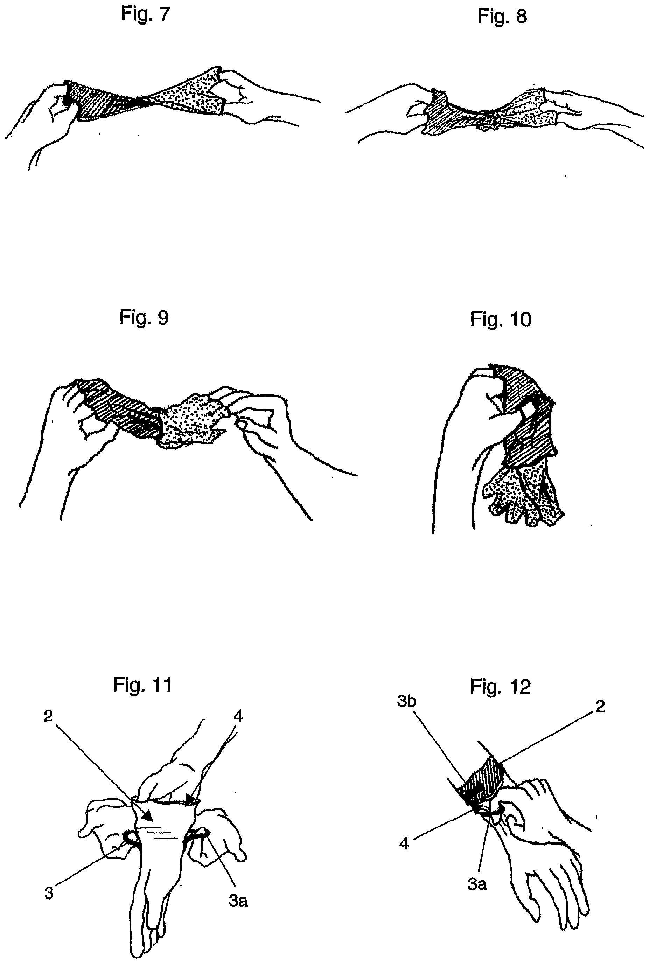

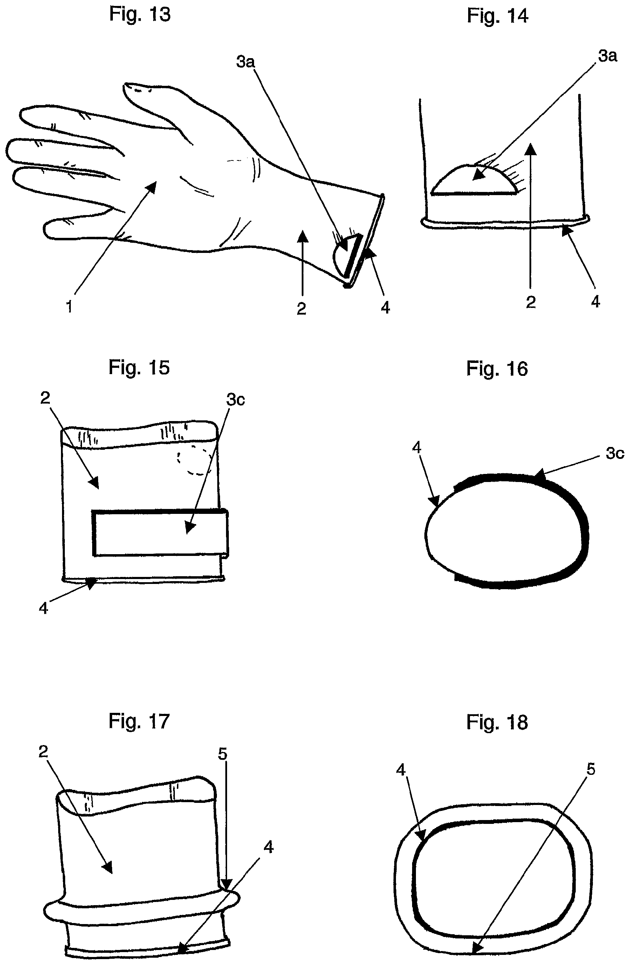

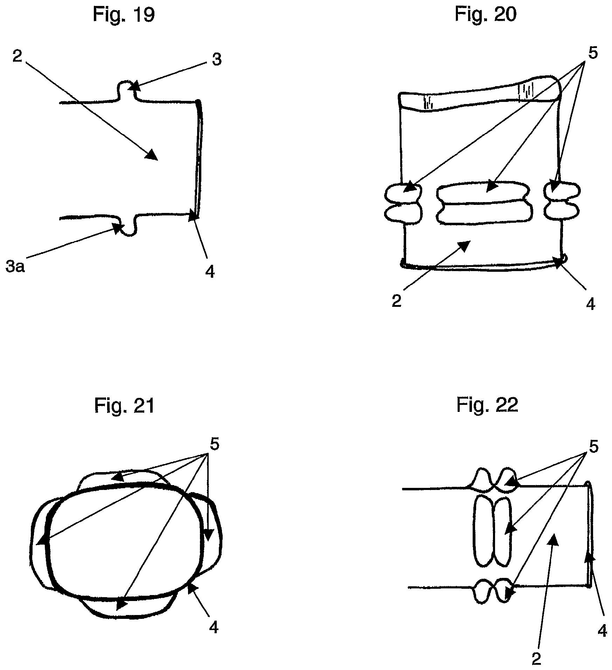

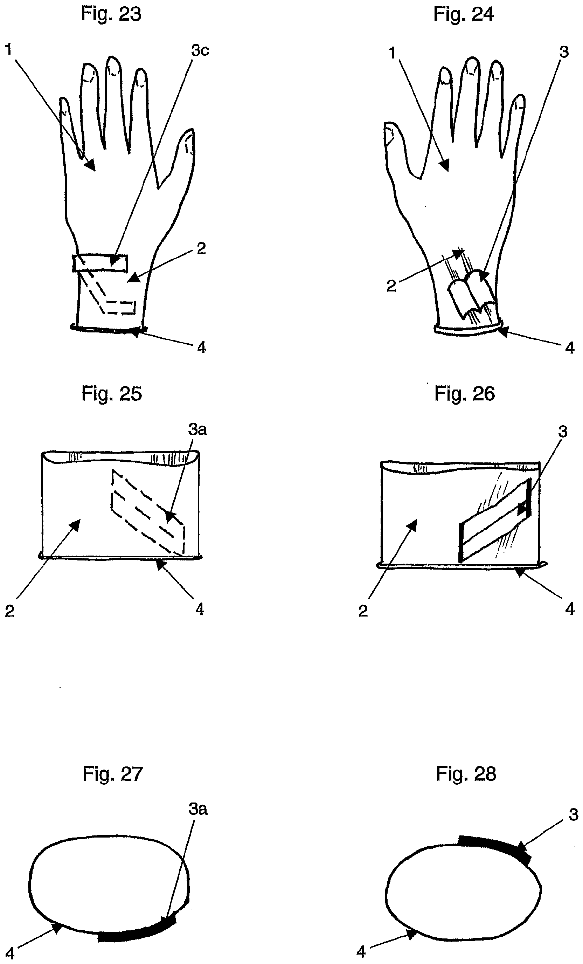

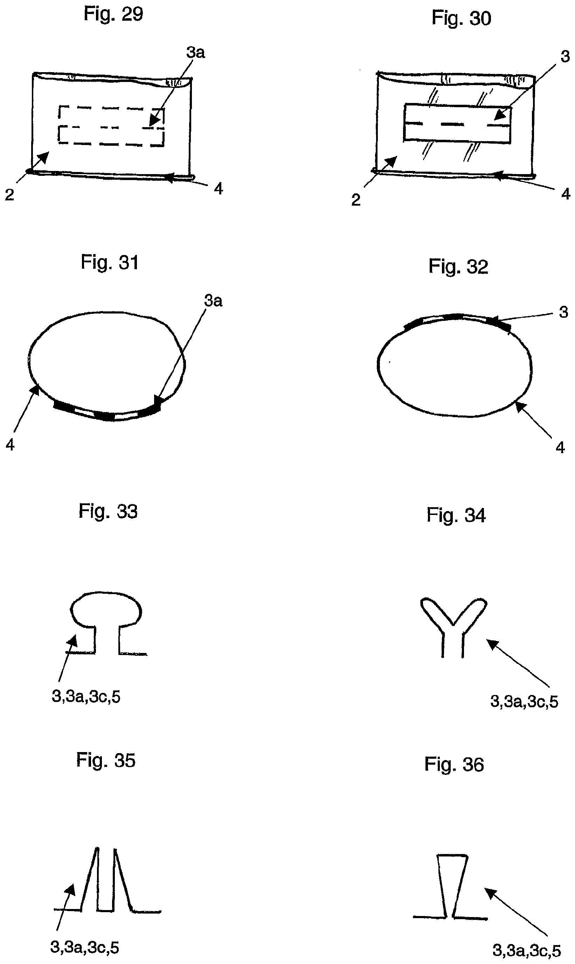

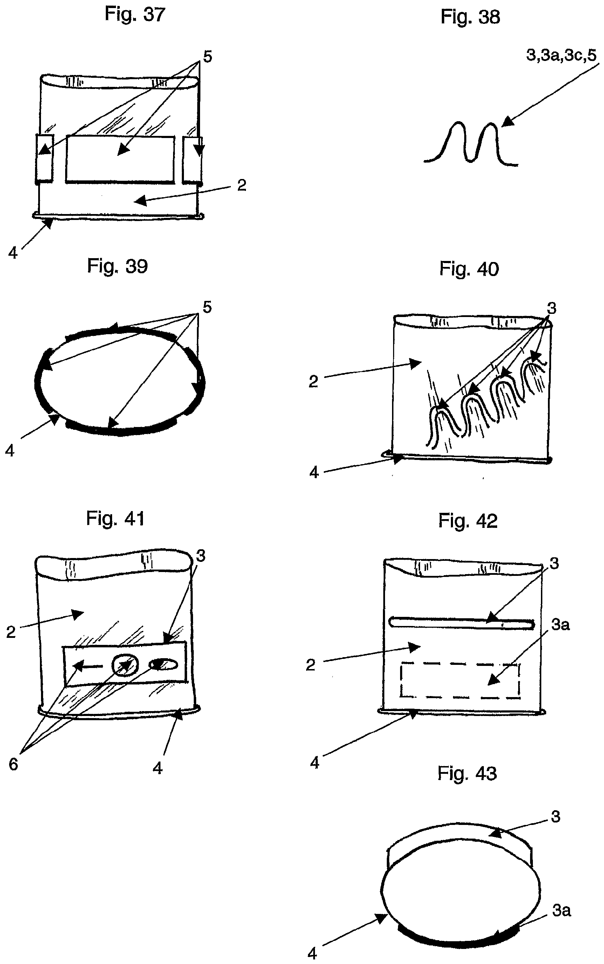

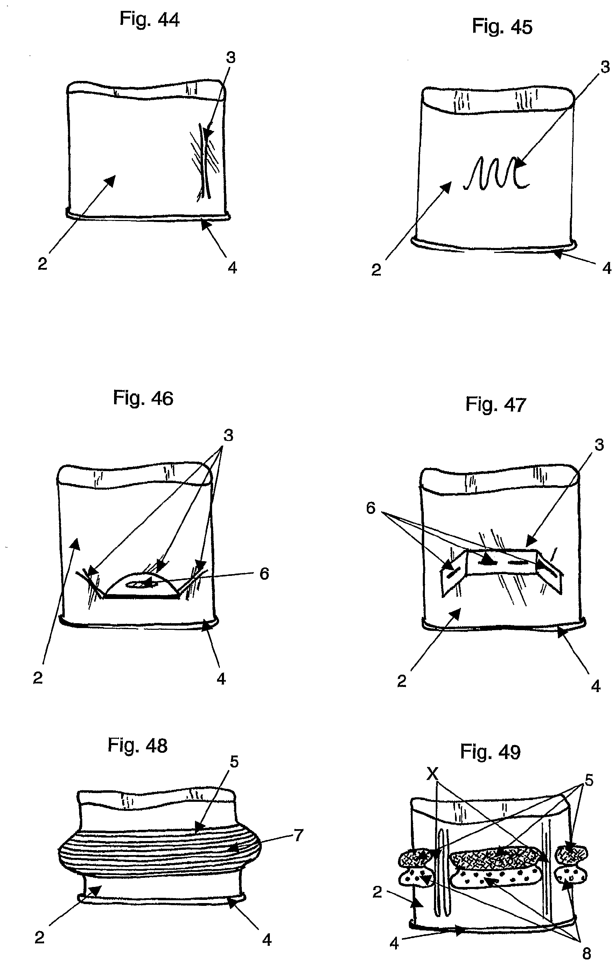

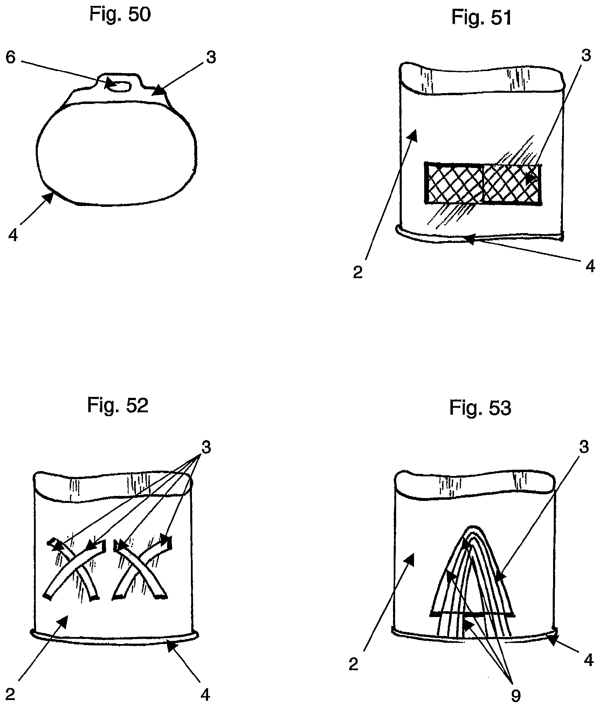

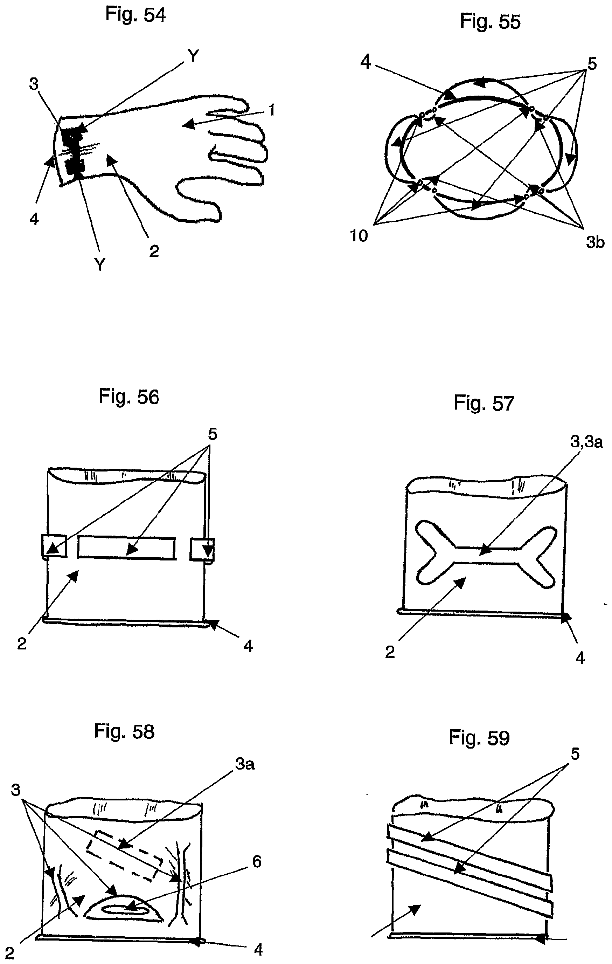

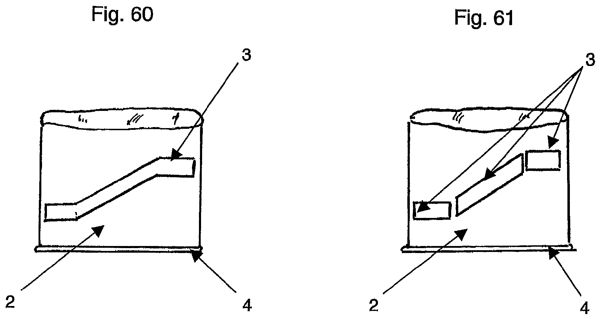

Dabei zeigt die Fig. 1 einen Handschuh mit einer einzigen laschenförmigen Greifhilfe, Fig. 2 ist eine Darstellung des Stulpenbereiches eines Handschuhs mit ebenfalls laschenförmiger Greifhilfe in vergrößertem Massstab, Fig. 3 bis 10 zeigen den gegengleichen Ausziehvorgang mit einem erfindungsgemäßen Handschuh-Paar in perspektivischer Darstellung, Fig. 11 zeigt ein Stadium beim Anziehen eines Handschuhs einer weiteren Ausführungsform mit zwei Greifhilfen, Fig. 12 zeigt ein Stadium beim Ausziehen eines Handschuhs einer weiteren Ausführungsform mit zusätzlicher innenliegender Greifhilfe, Fig. 13 zeigt eine Greifhilfe in einer anderen Position an der Stulpe, Fig. 14 ist eine Darstellung der Stulpe der Fig. 13 in größerem Massstab, Fig. 15 ist eine Darstellung einer sich über einen größeren Umfang erstreckenden Greifhilfe, Fig. 16 ist eine Ansicht des Handschuhs der Fig. 15 von hinten, Fig. 17 zeigt eine Stulpe mit sich stegförmig über den gesamten Umfang erstreckender Greifhilfe, Fig. 18 ist eine Ansicht des Handschuhs der Fig. 17 von hinten, Fig. 19 zeigt eine Stulpe eines Handschuhs in Seitenansicht, mit zwei einander im wesentlichen diametral gegenüberliegenden Greifhilfen, Fig. 20 zeigt eine Stulpe mit Greifhilfen gemäß einer weiteren Ausführungsform entlang des gesamten Umfangs, Fig. 21 ist eine Ansicht des Handschuhs der Fig. 20 von hinten, Fig. 22 zeigt eine Stulpe mit Greifhilfen ähnlich der Fig. 20 in Seitenansicht, Fig. 23 zeigt in der Draufsicht den linken Handschuh eines Handschuhpaares mit einer Greifhilfe, Fig. 24 zeigt einen rechten Handschuh, mit einer weiteren Variante einer Greifhilfe, Fig. 25 zeigt eine Stulpe eines linken Handschuhs eines Handschuhpaares in Draufsicht mit an der Unterseite angeordneter, gemäß einer weiteren Variante ausgeführten Greifhilfe, Fig. 26 zeigt die Stulpe des rechten Handschuhs des Handschuhpaares mit an der Knöchelseite oben angeordneter Greifhilfe, Fig. 27 und 28 zeigen die Stulpen der Fig. 25 und 26 in einer Ansicht von hinten, Fig. 29 und Fig. 30 zeigen jeweils eine Draufsicht auf Stulpen des linken bzw. rechten Handschuhs eines Handschuhpaares mit anders gestalteten Greifhilfen, Fig. 31 und 32 sind Ansichten der Handschuhe der Fig. 29 und 30 von hinten, Fig. 33 bis 36 zeigen unterschiedliche Arten von Greifhilfen in Seitenansicht, Fig. 37 zeigt eine umlaufende, jedoch unterbrochene Greifhilfe in Taschenform, Fig. 38 zeigt eine weitere Ausführungsform einer Greifhilfe in Seitenansicht, Fig. 39 ist eine Ansicht der Stulpe des Handschuhs der Fig. 37 von hinten, Fig. 40 zeigt einen Handschuh mit einer Reihe von nebeneinanderliegenden Greifhilfen, Fig. 41 ist eine Draufsicht auf eine Stulpe mit wieder anderer Ausführungsform der Greifhilfe, Fig. 42 zeigt eine Stulpe mit zwei Greifhilfen in unterschiedlicher Ausfüh- rungsform und diametral gegenüberliegend, Fig. 43 zeigt den Handschuh der Fig. 42 in Ansicht von hinten, Fig. 44 ist eine Ansicht einer Stulpe mit laschenförmiger Greifhilfe parallel zur Längsachse der Stulpe, Fig. 45 zeigt eine Stulpe mit fadenförmiger Greifhilfe, Fig. 46 ist eine Ansicht einer Stulpe mit umfangsmäßig aufeinanderfolgenden drei Greifhilfen unterschiedlicher Ausgestaltung, Fig. 47 zeigt ebenfalls eine Stulpe mit einer Greifhilfe mit unterschiedlicher Ausrichtung an verschiedenen Umfangspositionen, Fig. 48 ist die Ansicht einer Stulpe mit umlaufender Greifhilfe mit die Griffigkeit erhöhender Oberflächenstruktur, Fig. 49 zeigt eine Stulpe mit Greifhilfen mit Oberflächenstrukturen zur Erhöhung der Griffsicherheit und mit Dehnungsbereichen zwischen den einzelnen Greifhilfen, Fig. 50 zeigt die Hinteransicht eines Handschuhs mit einer wieder anderen Ausführungsform einer Greifhilfe, Fig. 51 ist eine Draufsicht auf eine Stulpe mit anderer Ausführungsform der Greifhilfe, Fig. 52 zeigt eine Stulpe mit einander übergreifenden, unterschiedlich ausgerichteten Greifhilfen in Laschenform, Fig. 53 zeigt eine Greifhilfenvariante mit Verstärkungsfasern, Fig. 54 zeigt eine Ansicht einer Stulpe mit separat gefertigter und dann aufgebrachter, laschenförmiger Greifhilfe, die Fig. 55 und 56 sind eine Hinteransicht und eine Draufsicht auf eine Handschuhstulpe mit einer weiteren Ausführungsform einer umlaufenden Greifhilfe, Fig. 57 zeigt eine weitere Ausführungform einer Greifhilfe, Fig. 58 ist eine Darstellung einer Stulpe mit einer Gruppe von aneinander anschließenden Greifhilfen, Fig. 59 zeigt eine weitere Ausführungsform für umlaufenden Greifhilfen und die Fig. 60 und 61 sind Darstellungen von Greifhilfen mit verschiedener Ausrichtung, einmal unmittelbar ineinander übergehend, dann mit kurzem Abstand aneinander anschließend.

Die Fig. 1 zeigt in der Draufsicht von oben einen rechten Handschuh, bestehend aus einem einheitlichen Handschuhkörper H, wobei der Handschuhkörper einen eigentlichen Handteil 1 und eine daran anschließende Stulpe 2 aufweist. Die Ausgestaltung des Handschuhs H findet hauptsächlich Verwendung bei dicht und unmittelbar an der Hand des Trägers anliegenden Varianten, vorzugsweise aus flüssigkeitsdichtem, elastischem Material, beispielsweise Gummi, welche vorzugsweise als Untersuchungs- oder Operationshandschuhe dienen. Nichtsdestotrotz erstrecken sich die Vorteile der Handschuhausführung auch auf alle anderen Handschuharten, wie auch Stoff-oder Lederhandschuhe oder auch alle Arten von Einweghandschuhen aus Plastik, wie in Supermärkten und an Tankstellen üblich.

An der Stulpe 2 ist nun außen an einem Umfangsabschnitt eine hier beispielsweise laschenförmige Greifhilfe 3a vorgesehen. Diese zumindest eine Greifhilfe 3a ist auf einem Umfangsteil der Stulpe 2 vorgesehen, welcher aussen den Knöchelbereich der Hand bedeckt, wobei diese Angabe aber lediglich als ungefähre Positionierung in Umfangsrichtung gesehen werden muss. Auch kann sich die Greifhilfe 3a über einen gewissen Winkelbereich im umfangsmäßigen Knöchelbereich erstrecken. In Längsrichtung gesehen kann die Greifhilfe 3a an sehr unterschiedlichen Positionen vorgesehen sein, für Handschuhe mit sehr kurzen Stulpen 2 sogar in den Bereich des eigentlichen Handteils 1 wandern und im Bereich des Handgelenks oder sogar der Handkante liegen. Diese und alle weiteren noch zu erläuternden Greifhilfen sind vorteilhafterweise vom Handschuhkörper H so wenig entfernt bzw. stehen so wenig davon ab, dass sie einerseits leicht und sicher zu ergreifen sind, jedoch in keiner Weise bei Arbeits- und Untersuchungsvorgängen etc. stören. Auch sind sie derart weit vom hinteren Rand 5 der Stulpe 2 beabstandet, dass keine Gefahr besteht beim An- bzw. Ausziehvorgang mit der einen behandschuhten Hand das Gewebe (Haut) der gegenüberliegenden Hand zu berühren.

Fig. 2 zeigt die Greifhilfe 3 in größerem Maßstab, wobei erkennbar ist, dass diese Greifhilfe 3 einstückig mit der Handschuhstulpe 2 ausgeführt ist und sich derart über die der Haut des Trägers entgegengesetzte Oberfläche der Stulpe 2 erhebt, dass ein oder sogar mehrere Finger der anderen Hand oder auch ein Hilfsmittel zum Handhaben des Handschuhs hindurchgesteckt werden können. Bei diesem Hilfsmittel kann es sich etwa um Haken oder dgl. handeln, die fest angebracht sein oder mit der anderen Hand gehalten werden können.

Jede Art von Greifhilfe 3 kann in beliebiger sinnvoller Ausrichtung in Bezug auf die Längsachse der Stulpe 2 vorgesehen sein.

Die Fig. 2 zeigt eine schräge Anordnung beispielsweise einer laschenförmigen Greifhilfe ähnlich der Fig. 1, welche optimal die Ausübung einer Zugkraft vom Knöchelbereich des Handschuhs schräg über die Handrückenseite hin zur Daumenseite gestattet.

Fig. 3 zeigt den Beginn des Ausziehvorganges eines Paares erfindungsgemäßer Handschuhe in perspektivischer Ansicht. Neben der bereits in Zusammenhang mit Fig. 1 und Fig. 2 beschriebenen Greifhilfe 3 im Knöchelbereich der Stulpe 2 ist vorzugweise am Handschuh H der anderen Hand eine weitere Greifhilfe 3 vorgesehen, die in Umfangsrichtung gesehen an einer dem Handrückenteil 4 gegenüberliegenden Position vorgesehen ist, oder an einer Stelle, die sich auf zumindest einer Seite daran anschließt. Die erste Greifhilfe wie auch die weitere Greifhilfe 3 könnten auch als sich über etwa 180° erstreckende, vom Knöchelbereich auf die Handinnenseite reichende Strukturen ausgeführt sein.

Nach Ergreifen der Greifhilfe 3 am in der Fig. 3 linken Handschuh mit der anderen, d. h. rechten, Hand (Fig. 3) kann, wie in Fig. 4 dargestellt ist, die im Knöchelbereich an der Außenseite des zweiten, rechten Handschuhs angeordnete Greifhilfe 3 von der linken Hand in einer ergonomisch günstigen Stellung beider Hände bzw. Unterarme zueinander ergriffen werden. Die Haut des Trägers kommt mit keiner verschmutzten oder kontaminierten äußeren Oberfläche der Handschuhe, in Kontakt. Beim weiteren Auseinanderziehen der Hände, wie es in den Fig. 5 und 6 zu sehen ist, werden dann gegengleich und im wesentlichen gleichzeitig die Handschuhe, von den Unterarmen und dann auch der Handfläche bzw. dem Handrücken abgezogen, wobei die Handschuhe auch bereits zum Teil mit ihrer normalerweise außen liegenden Oberfläche in den jeweils anderen Handschuh hineingezogen werden. Beim weiteren Auseinanderziehen der Hände und dem Abziehen der Handschuhe auch noch von den Fingern beider Hände werden die Handschuhe schliesslich ganz gewendet und durch das weite Ineinanderziehen miteinander verbunden, wie in den Fig. 7 bis 10 zu erkennen ist. Der Benutzer kann daher, wie in Fig. 10 zu erkennen ist, die Handschuhe nach dem Ausziehen halten und handhaben, ohne mit deren allenfalls verschmutzter oder kontaminierter Aussenseite in Kontakt zu kommen.

Die erfindungsgemäß ausgestaltete und angeordnete Greifhilfe 3 erleichtert nicht nur wie oben erläutert den Ausziehvorgang, sondern der Benutzer kann den Handschuh selber leichter überstreifen, indem er ihn an einer Lasche od. dgl. ergreift und ihn durch Ziehen daran in Richtung Arm bzw. Körper überstreift. Das Gleiche kann auch, wie in Fig. 11 zu sehen ist, ein Helfer machen, der eventuell schon (sterile) Handschuhe trägt, wobei dann weitgehende Sterilität gewährleistet ist. Ausserdem kann diese zweite Person den Handschuh aufdehnen indem sie, wenn der Handschuh zwei oder mehrere Greifhilfen 3, 3a hat, diese auseinanderzieht und so die Öffnung des Handschuhs H aufdehnt. Der Benützer des Handschuhs kann dann leichter in die aufgedehnte Handschuhöffnung (und den Handschuh) schlüpfen.

Wenn die Stulpe 2 des Handschuhs H bereits in irgendeiner Weise ein Stück umgeschlagen ist, wenn allenfalls die außen liegenden Greifhilfen 3, 3a verloren wurden oder durch die Finger gerutscht sind, kann der weitere Ausziehvorgang auch erleichtert werden, wenn zusätzlich zumindest eine Greifhilfe 3b innen in der Stulpe 2 vorgesehen ist, wie dies in Fig. 12 gezeigt ist. Vorzugsweise wird eine derartige Greifhilfe 3b innen in der Stulpe 2 näher dem Handteil 1 des Handschuhs H liegen als eine Greifhilfe 3, 3a außen auf der Stulpe 2.

Andere Arten von Greifhilfen 3, 3a, 3b, deren Ausgestaltungen, Anordnungen und Orientierungen, sind dann beispielhaft aber nicht beschränkend in den Fig. 13 bis 54 dargestellt.

Neben Greifhilfen 3, 3a in der zuvor beschriebenen Ausführungsform als Lasche mit einer durchgehenden Öffnung können auch Greifhilfen 3, 3a in zungen- oder lappenförmiger Ausbildung vorgesehen sein, wie dies in Fig. 13 und 14 für eine an der Handinnenseite angeordnete Greifhilfe 3 dargestellt ist. Diese lappenförmige Greifhilfe 3 liegt normalerweise flach an der Oberfläche der Stulpe 2 auf, wie dies auch für die laschenförmigen und alle weiteren Ausführungsformen von Greifhilfen 3, 3a, 3b vorteilhafterweise vorgesehen ist.

In Fig. 15 ist in der Draufsicht eine über einen weiten Umfangsbereich umlaufende Greifhilfe 3 dargestellt, die sich um den eigentlichen Knöchelbereich noch auf die Handrückenseite und auch die Handunterseite der Stulpe 2 erstreckt, wie in der Ansicht von hinten in Fig. 16 deutlich zu erkennen ist. Die Greifhilfe 3 der Fig. 15 und 16 ist auch vom Aufbau her anders als die bisher erläuterten Greifhilfen 3, 3a, 3b, nämlich in Form einer nach vorne, d. h. zum Handteil 1 des Handschuhs H geschlossenen und nach hinten hin offenen Tasche ausgeführt. Auch Ausführungen mit zentralem Steg und somit nach vorne als auch hinten offener Taschenform sind möglich, in welche die Finger der anderen Hand oder auch andere Hilfsmittel zum An- und Ausziehen von vorne und/oder von hinten hineingreifen und so die Greifhilfe sicher halten können.

Eine weitere Ausführungsform der Greifhilfe, welche nicht Gegenstand der Erfindung ist, ist in den Fig. 17 und 18 zu erkennen, wo eine den gesamten Umfang der Stulpe 2 umlaufende Erhebung 5 als Greifhilfe vorgesehen ist. Diese Erhebung 5 kann durch eine Ausbuchtung des Materials der Stulpe 2 selbst gebildet sein oder durch zusätzliches Material, das auf die Stulpe 2 aufgebracht ist. Für die Herstellung der Ausbuchtung - wie für jegliche Art von Greifhilfen in Form von Ausbuchtungen - kann die Tauchform für Handschuh mit einer entsprechenden Ausformung versehen sein. Diese Ausformung kann nach Mittauchen und Fertigstellung des Handschuhs auf der Tauchform verbleiben, aus dem Handschuh entfernt werden oder auch allenfalls darin verbleiben. Die Tauchform kann auch eine permanente Ausbuchtung haben.

Selbstverständlich können für Handschuhpaare wie auch für Einzelhandschuhe die Stulpe 2 vollständig umrundende Greifhilfen 4 vorgesehen sein. Auch könnten diese umlaufende Greifhilfen 4 taschenförmig ausgebildet sein, sowohl mit nach hinten, d. h. zum hinteren Rand 5 der Stulpe 2 weisender, Öffnung als auch nach vorne offen oder abwechselnd in beide Richtungen offen.

Fig. 19 zeigt andere Greifhilfen 3, 3a in Ausbuchtungs-Ausführung, welche als im wesentlichen kreissymmetrische Erhebungen der Stulpe 2 ausgeführt und gegen das Handschuh-Innere hohl und offen sind. Letztere Ausführungsform kann auch für die umlaufende Erhebung 5 der Fig. 17 und 18 vorgesehen sein.

Eine den Fig. 17 und 18 ähnliche Ausführungsform der Greifhilfe ist in den Fig. 20 und 21 dargestellt. Zwei dicht in Richtung der Längsachse der Stulpe 2 nebeneinanderliegende und im wesentlichen senkrecht auf diese Achse orientierte Grate bilden eine mit Ausnahme von vier Unterbrechungen die Stulpe 2 komplett umrundende Greifhilfe 5. Die Grate der Greifhilfe 5 reichen im wesentlichen über einen Winkelbereich von ca. 45° und befinden sich - umfangsmäßig gesprochen - auf der Handrückenseite, dem äußeren Knöchelbereich, der Daumenseite und der Handinnenseite der Stulpe 2.

Fig. 22 zeigt eine Stulpe 2 mit Greifhilfen 5 ähnlich der Fig. 20 in Seitenansicht, wieder als zwei nebeneinanderliegende und durch vier Unterbrechungen in vier getrennte Bereiche geteilte Grate ausgeführt. Während die Fig. 20 jedoch eine Ausführungsform darstellt, bei welcher die Greifhilfe 5 durch zusätzlich auf die Stulpe 2 aufgebrachtes Material gebildet ist, sind die Grate gemäß Fig. 22 durch Ausstülpungen der Stulpe 2 selbst, vorzugsweise nach innen hin offene, vorzugsweise während des Tauchvorganges bei der Herstellung ausgebildete Hohlräume, gebildet.

Die Greifhilfe 3 des linken Handschuhs eines Handschuhpaares verläuft auf der Handrückenseite des Knöchelbereiches im wesentlichen parallel zum hinteren Rand 4 der Stulpe 2, geht dann einen Abschnitt über, der schräg in Richtung dieses hinteren Randes 4 zur Handinnenseite verläuft und von dort wieder ein kleines Stück weitgehend parallel zum Rand 4 verläuft.

Die im oberen Knöchelbereich des rechten Handschuhs eines Handschuhpaares vorgesehene Greifhilfe 3 ist in Form von zwei Halbröhren ausgeführt, die gegenüber der Achse der Stulpe 2 schräg, in Richtung von der Knöchelseite auf die Daumenseite hin laufend, orientiert sind. Der Benutzer kann mit den Fingern der anderen Hand und/oder einem Hilfswerkzeug von beiden Seiten her in die halbröhrenförmige Greifhilfe hineinschlüpfen.

Fig. 25 zeigt eine Stulpe eines linken Handschuhs eines Handschuhpaares wie in den Fig. 23 und 24 in der Draufsicht. Als Greifhilfe 3 ist - durch strichlierte Darstellung angedeutet - an der Handinnenseite der Stulpe 2 und umfangsmässig im Daumenbereich, d. h. der Unterseite in Fig. 25, eine längliche, nach vorne und hinten hin offene Tasche angebracht.

Die Längsachse der als Greifhilfe 3 dienenden Tasche, und in gleicher Weise ihr zentraler, mit der Stulpe 2 verbundener Innensteg, ist gegenüber der Längsachse der Stulpe 2 geneigt, schließt hier beispielsweise einen Winkel von ca. 40 mit dieser Längsachse der Stulpe 2 ein.

Die Greifhilfe 3 kann - in jeder ihrer Ausführungsvarianten - in einem derartigen Winkel zur Handschuhachse angeordnet sein, auch deutlich von einer normal auf die Längsachse orientierten Anordnung abweichend, dass zumindest ein Finger der gegenüberliegenden Hand ergonomisch, leicht und sicher die Greifhilfe erfassen und halten kann.

Fig. 26 zeigt die Stulpe des rechten Handschuhs des Handschuhpaares mit an der oberen Knöchelseite angeordneter Greifhilfe 3, die gleich der Greifhilfe 3 der Fig. 25 als beidseitig offene Tasche ausgeführt ist. Fig. 27 und 28 zeigen die Stulpen der Fig. 25 und 26 in einer Ansicht von hinten, in welcher die umfangsmäßige Position der Greifhilfen als auch deren umfangsmäßige Erstreckung zu erkennen ist.

Eine andere Ausführungsform und auch Anordnung von Greifhilfen 3 und 3a eines zusammengehörigen Handschuhpaares - ähnlich den Paaren der Fig. 23 bis 28 - zeigen die Fig. 29 und 30. Wieder wird durch strichlierte Darstellung der Greifhilfe 3 in Fig. 29 symbolisiert, dass sich diese auf der Unterseite, umfangsmäßig der Handinnenseite der Stulpe 2, befindet, während die Greifhilfe 3a am gegenüberliegenden Handschuh der Fig. 30 auf der Handrückenseite der Stulpe 2 angebracht ist. Die Längsachse der Greifhilfen 3, 3a sind hier nun genau senkrecht auf die Längsachse des Handschuhs orientiert und die Greifhilfen 3, 3a erstrecken sich über einen umfangsmäßigen Winkelbereich von ca. 90° der Stulpe 2. Wie besonders deutlich in den Ansichten von hinten der Fig. 31 und 32 zu erkennen ist, sind die Greifhilfen 3, 3a beispielhaft durch vorne und hinten offene Taschen gebildet, bei welchen aber der zentrale Steg zwei Unterbrechungen aufweist, wodurch zwei Öffnungen gebildet sind, durch welche ein Finger der anderen Hand oder auch ein Hilfswerkzeug zum noch besseren Ergreifen der Greifhilfen 3, 3a hindurchschlüpfen kann.

Dem besseren Ergreifen der Greifhilfen 3, 3a können auch bestimmte Querschnittsformen dienen. So zeigt die Fig. 33 eine dem griechischen Buchstaben "Omega" ähnliche Form einer Greifhilfe, die allenfalls auch in eine pilzförmige Ausführung mit nach unten abgebogenem Aussenrand übergehen könnte. Auch die ähnliche Querschnittsverbreiterung nach oben hin der Greifhilfe der Fig. 34, in Form des Buchstaben "Y", erleichtert das sichere Halten der Greifhilfe. Eine andere Variante der Greifhilfe ist in Fig. 35 dargestellt, weiche im Querschnitt bzw. der Seitenansicht zwei Dreiecken entspricht, die eine einander zugewandte, parallele Seite aufweisen. Ein Greifhilfen-Querschnitt in Form abgerundeter, nebeneinanderliegender Grate ist in Fig. 38 gezeigt. Bevorzugt sind jedoch sich nach oben verbreiternde Formen, wie etwa Greifhilfen mit dem beispielsweise in Fig. 36 gezeigten Querschnitt bzw. Seitenansicht in Form eines kopfstehenden Dreiecks.

Für manche Anwendungszwecke könnten allenfalls auch nach vorne offene Taschen als Greifhilfe vorteilhaft sein, wie beispielsweise bei der in Fig. 37 und 39 dargestellten Ausführungsform eines Handschuhs. Wie die Ansicht der Stulpe 2 von hinten in der Fig. 39 deutlich macht, kann auch eine taschenförmige Greifhilfe 5 im wesentlichen die Stulpe 2 umfangsmäßig vollständig umlaufend und nur durch kleine Unterbrechungen in separate Bereiche unterteilt ausgeführt sein. Die einzelnen taschenförmigen Bereiche erstrecken sich winkelmäßig über etwas mehr als 90 , sind am Handrückenbereich, an der Handinnenseite, im Knöchelbereich und im Daumenbereich des Umfangs der Stulpe 2 positioniert und sind durch schmale greifhilfefreie Stulpenbereiche voneinander getrennt.

Wie in Fig. 40 zu erkennen ist, können beliebig viele einzelne Greifhilfen bzw. Greifhilfenabschnitte aneinander anschließend, mit oder ohne Abstand zueinander, vorgesehen sein.

Beispielhaft ist dies in Fig. 40 anhand von vier in einer schrägen Linie im oberen Knöchelbereich der Stulpe 2 angeordneten Einzellaschen dargestellt, von welchen jede geringfügig von der benachbarten Lasche beabstandet ist und wobei jede Lasche selbst in Richtung vom Knöchelbereich auf die Daumenseite der Hand ausgerichtet ist, um in dieser Richtung optimal Zug ausüben zu können.

Fig. 41 ist eine Draufsicht auf eine Stulpe mit wieder anderer Ausführungsform der Greifhilfe 3a auf der Handrückenseite der Stulpe 2. Eine taschenförmige Greifhilfe 3a mit senkrechter Orientierung auf die Längsachse des Handschuhs ist zusätzlich mit Öffnungen 6 in jenem Bereich versehen, welcher die Oberseite der Tasche bildet. Diese Öffnungen 6 können beispielsweise in Form von Schlitzen (ganz links), im wesentlichen kreisförmigen (mittig) oder auch elliptischen Löchern (ganz rechts) vorliegen, wobei bei mit einer Vorzugsrichtung versehenen Öffnungen 6 diese Vorzugsrichtung in prinzipiell beliebiger Orientierung relativ zur Längsachse der Greifhilfe 3a und/oder der Stulpe 2 des Handschuhs vorliegen kann.

Wie als weiteres Ausführungsbeispiel in Fig. 42 dargestellt, können an einem Handschuh natürlich auch zwei oder mehr verschiedene Ausführungen von Greifhilfen 3, 3a kombiniert sein, ebenso wie verschiedene Positionen und Ausrichtungen der Greifhilfen relativ zueinander und/oder zur Längsachse des Handschuhs. Im Beispiel der Fig. 42 ist auf der Handrückenseite der Stulpe 2 eine gerade, senkrecht zur Längsachse des Handschuhs orientierte gratförmige Erhebung als Greifhilfe 3 und diametral gegenüberliegend auf der Handinnenseite eine taschenförmige Greifhilfe 3a, ebenfalls senkrecht auf die Längsachse der Stulpe 2 stehend, vorgesehen. Die umfangsmäßige Position ist deutlich in Fig. 43 dargestellt.

Mehrere Greifhilfen 3, 3a können selbstverständlich nicht nur auf unterschiedlichen Handschuhen, sondern auch auf ein und demselben Handschuh in Längsrichtung des Handschuhs unterschiedliche Positionen einnehmen. So ist im Ausführungsbeispiel der Fig. 42 die gratförmige obere Greifhilfe 3 weitere vom hinteren Rand 4 der Stulpe 2 beabstandet als die taschenförmige untere Greifhilfe 3a, die weiter vom Handteil 1 des Handschuhs entfernt liegt.

In jedem Fall ist aber auch die dem hinteren Rand 4 nächstliegende Greifhilfe 3, 3a oder 5 soweit vom hinteren Rand 4 der Stulpe 2 beabstandet, dass keinerlei Gefahr besteht beim Ergreifen der Greifhilfe 3, 3a oder 5 mit einem verschmutzten oder kontaminierten Handschuh die nicht mehr von der Stulpe 2 bedeckte Haut oder Kleidung des Trägers zu berühren.

Fig. 44 ist eine Ansicht einer Stulpe 2 mit laschenförmiger Greifhilfe 3, welche parallel zur Längsachse der Stulpe 2 orientiert ist.

Eine ganz andere Ausführungsform einer Greifhilfe 3 zeigt die Fig. 45. Sie ist als im Ruhezustand schlangenförmig an der Stulpe 2 anliegender Faden ausgeführt, der mit der anderen Hand von der Stulpe 2 abgehoben und zum Ausüben eines Zuges in im wesentlichen beliebiger Richtung verwendet werden kann.

Mehrere, auch unterschiedlich ausgeführte, Greifhilfen können auch unmittelbar aneinander anschließen, wie das in Fig. 46 beispielhaft dargestellt ist. Hier sind zu beiden Seiten einer zentralen, laschen- bzw. zungenförmigen Greifhilfe mit zusätzlich einem Langloch für sichereres Ergreifen laschenartig ausgebildete Greifhilfen angeordnet. Die unterschiedlichen, aneinander anschließenden Greifhilfen 3 könnten gleich oder parallel orientiert sein, sie können aber auch wie dargestellt unterschiedliche Ausrichtung zueinander aufweisen.

Beispielhaft dafür zeigt die Fig. 46 eine Ausführungsform, bei welcher die äußeren Ende der außenliegenden, laschenförmigen Bereiche der Greifhilfe 3 zum Handteil 1 des Handschuhs hin versetzt sind.

Fig. 47 zeigt eine Stulpe mit einer Greifhilfe 3 mit unterschiedlicher Ausrichtung an verschiedenen Umfangspositionen, wobei die verschiedenen Bereiche der Greifhilfe 3 jedoch im wesentlichen gleichartig ausgeführt sind, nämlich als taschenförmige Struktur mit Schlitzen in der oberen Taschenbegrenzung. Im Gegensatz zur Greifhilfe 3 der Fig. 46 sind hier aber die äußeren Ende der außenliegenden Abschnitte der Greifhilfe 3a in Richtung auf den hinteren Rand 4 der Stulpe 2 hin versetzt.

Um das Ergreifen und das Halten der Greifhilfen während des An- oder Ausziehvorganges zu erleichtern, können verschiedene Maßnahmen zur Erhöhung der Griffigkeit der Oberfläche im Bereich der Greifhilfen vorgesehen sein. So können, wie in Fig. 48 beispielhaft dargestellt ist, Rillen 7 vorhanden sein, welche bei länglichen Greifhilfen und auch im Fall von umlaufenden Greifhilfen 3 in Längsrichtung bzw. in Umfangsrichtung der Stulpe 2 verlaufen. Diese Rillen 7 können auch durch Falten des Materials der Stulpe 2 ausgebildet sein, was gleichzeitig die Aufweitung des Bereiches der Greifhilfe 3 gestattet, die damit noch leichter und einfacher gehalten werden kann.

Neben Rillen 7 wie in Fig. 48 sind auch anders strukturierte Oberflächenbereiche für die Greifhilfen 5 möglich. Neben einfach aufgerauten Flächen können auch Noppen 8 oder andere Erhebungen flächig vorgesehen sein. Die Noppen 8 selbst können verschiedenste Ausbildungen aufweisen, wie beispielsweise im wesentlichen halbkreisförmig, zapfenförmig, kegel- oder pyramidenförmig, usw. Auch können unterschiedliche Ausbildungen in aneinandergrenzenden Bereichen vorgesehen sein, wie dies beispielhaft für die umlaufende, unterbrochene Greifhilfe 5 der Fig. 49 dargestellt ist. In Fig. 49 ist auch noch zusätzlich dargestellt, dass nicht nur die Greifhilfe 5 selbst (wie in Fig. 48) sondern dass auch die Zwischenbereiche X mit dehnungsfähiger Ausführung wie Rillen oder Falten oder dehnungsfähigem Material ausgestattet sein können.

Eine sich etwas über die Oberfläche der Stulpe 2, hier beispielhaft auf der Handrückenseite, erhebende Greifhilfe 3 ist Fig. 50 gezeigt. Sie besteht aus einem lappenförmigen Teil, dessen zentraler Bereich höher ausgeführt ist als die Seitenteile und der zusätzlich noch mit einem quer zur Stulpe 2 orientierten Langloch 6 versehen ist.

Fig. 51 zeigt in der Draufsicht eine weitere Ausführungsform einer Stulpe 2 mit einer taschenförmigen Greifhilfe 3, welche in einem Längsabschnitt nach hinten hin, auf den hinteren Rand 4 der Stulpe 2 hin, und im anschließenden (in der Zeichnung rechten) Abschnitt nach vorne hin, auf den Handteil 1 des Handschuhs hin, offen ist.

Zusätzlich ist zur Erhöhung der Griffigkeit die äußere Oberfläche der Greifhilfe 3 mit beispielsweise einer Waffelprägung versehen.

Dass die Greifhilfen 3, 3a, 3c, 5 gemäß der vorliegenden Erfindung nicht nur aneinander in Umfangsrichtung und auch in Längsrichtung der Stulpe 2 anschließen können, sondern einander auch überschneiden können, ist beispielhaft in Fig. 52 dargestellt. Hier sind auf der Handrückenseite der Stulpe 2 vier laschenartige Greifhilfen 3 vorgesehen, von welchen jeweils zwei Laschen einander kreuzförmig überschneidend angeordnet sind. Die einzelnen Laschen sind beispielhaft um jeweils im wesentlichen 45 gegenüber der Längsachse der Stulpe 2 geneigt und ebenfalls beispielhaft sind jeweils zwei kreuzförmige Laschenpaare nebeneinander in im wesentlichen gleichem Abstand vom hinteren Rand 4 der Stulpe 2 positioniert.

Fig. 53 zeigt eine zungenförmige, auf den Handteil des Handschuhs hin weisende Greifhilfe 3 am Handrückenteil der Stulpe 2, wobei in die Greifhilfe 3 selbst und den Verbindungsbereich Verstärkungsfasern eingelagert sind. Diese Art der Verstärkung des Verbindungs- bzw. Übergangsbereiches von Greifhilfe und Stulpe kann selbstverständlich für jede Ausführungsform von Greifhilfe und Stulpe vorgesehen sein.

Beispielhaft für separat gefertigte und dann auf die Stulpe 2 aufgebrachte Greifhilfen 3 ist in Fig. 54 eine laschenförmiger Greifhilfe 3 für die Handrückenseite des Handschuhs dargestellt, welche an ihren der Stulpe 2 zugewandten Enden plättchenartige Verbreiterungen 3c aufweist, mit welchen die Greifhilfe 3 mit der Stulpe 2 vorzugsweise verklebt werden kann.

Eine umlaufende Greifhilfe 5 kann aber auch gemäß der in den Fig. 55 und 56 dargestellten Ausführungsform aufgebaut sein. Dabei ist ein geschlossenes oder mit beiden Enden am Handschuh befestigtes Band an mehreren Stellen, hier an acht Stellen, durch die Stulpe 2 des Handschuhs hindurchgeführt, vorzugsweise abgedichtet oder an den Durchführungsstellen mit dem Material der Stulpe dicht verbunden, beispielsweise verklebt oder verschweißt. Dadurch entstehen an der Außenseite des Handschuhs mehrere, hier vier, Schlaufen als außenliegende Greifhilfen 3 und gleichzeitig könnten die innenliegenden Abschnitte des Bandes, hier ebenfalls vier, bei ausreichendem Abstand zwischen den Durchgangspunkten durch die Stulpe 2, als innere Greifhilfen 3b genutzt werden.

Fig. 57 zeigt eine weitere Ausführungform einer Greifhilfe 3, 3a, bei welcher nach außen hin verzweigte Randabschnitte durch einen geraden, parallel zum hinteren Rand 4 der Stulpe 2 liegenden Abschnitt verbunden sind.

Auf der Stulpe 2 der Fig. 58 ist eine Gruppe von aneinander anschließenden Greifhilfen 3, mit Abstand voneinander positioniert, auch beispielhaft mit unterschiedlicher Ausrichtung gegenüber der Längsachse der Stulpe 2 und in unterschiedlichen Ausführungsformen, d. h. zentral eine lappenförmige Greifhilfe 3 mit Langloch 6 und seitlich zwei laschenförmige Greifhilfen 3, dargestellt. Durch strichlierte Darstellung symbolisiert ist auch an der Handinnenseite der Stulpe 2 beispielhaft eine hier als zumindest einseitig offene Tasche ausgeführte Greifhilfe 3a vorgesehen.

Fig. 59 zeigt beispielhaft, dass auch umlaufende Greifhilfen 5 gruppenweise vorgesehen sein können. Weiters ist durch die Fig. 59 beispielhaft dargestellt, dass auch diese umlaufenden Greifhilfen 5 schräg gegenüber der Längsachse der Stulpe 2 orientiert sein können, allenfalls natürlich auch für jede der Greifhilfen 5 der Gruppe unterschiedlich.

Fig. 60 und 61 sind Darstellungen von Greifhilfen 3, die sich über einen größeren Winkelbereich vom äußeren Knöchelbereich über den Handrückenbereich der Stulpe 2 erstrecken und in ihrem Verlauf beispielhaft zweimal die Ausrichtung gegenüber der Längsachse der Stulpe 2 ändern. Während die Greifhilfe 3 der Fig. 60 aus drei Abschnitten besteht, die unmittelbar ineinander übergehen, zeigt die Fig. 61 eigentlich eine Gruppe mit beispielhaft drei einzelnen, unterschiedlich ausgerichteten und mit kurzem Abstand aneinander anschließenden Greifhilfen 3.

## Patentansprüche

1. Handschuhpaar, bestehend aus zwei Handschuhen, insbesonders Arbeits-, Untersuchungs- oder Operationshandschuhen, mit jeweils einem einheitlichen Handschuhkörper aus flüssigkeitsdichtem, elastischem Material, beispielsweise Gummi, wobei der Handschuhkörper einen eigentlichen Handteil (1) und eine daran anschließende Stulpe (2) aufweist, wobei an jedem Handschuh des Handschuhpaars an der äußeren Oberfläche der Stulpe (2) und beabstandet vom hinteren Rand (4) der Stulpe (2) an einem Umfangsabschnitt eine Greifhilfe (3, 3a, 5) vorgesehen ist, die Greifhilfe (3, 3a) jedes Handschuhs als zur Innenseite des Handschuhs offene, hohle Ausbuchtung des Handschuhs nach außen ausgebildet ist, und die Greifhilfe (3, 3a, 5) des einen Handschuhs des Handschuhpaars und die Greifhilfe (3, 3a, 5) des anderen Handschuhs des Handschuhpaars auf aneinander angrenzenden Umfangsabschnitten der Stulpe (2) vorgesehen sind und einen Winkelabstand von zumindest 135° aufweisen, wobei die Greifhilfe des einen Handschuhs an der Handinnenseite angeordnet ist, **dadurch gekennzeichnet, dass** die Greifhilfe des anderen Handschuhs des Handschuhpaars an der Handrückenseite angeordnet ist, sodass die Greifhilfe jedes Handschuhs in der Stellung der gegengleich parallel gehaltenen Arme und Hände von der jeweils anderen Hand ergriffen und gleichzeitig und gegengleich ausgezogen und gleichzeitig automatisch gewendet werden, ohne dass die verschmutzte oder kontaminierte Außenseite der Handschuhe mit der Haut des Trägers in Kontakt kommt, und dass die Handschuhe des Handschuhpaars durch das weitere Ineinanderziehen miteinander verbunden werden.

2. Handschuhpaar nach Anspruch 1, **dadurch gekennzeichnet, dass** die Greifhilfe (3, 3a, 5) auf beiden Handschuhen im Wesentlichen diametral gegenüberliegend angeordnet sind.

3. Handschuhpaar nach Anspruch 1 oder 2, **dadurch gekennzeichnet, dass** sich die Greifhilfe (3, 3a, 5) jedes Handschuhs über einen Winkelbereich von zumindest 90° erstreckt.

4. Handschuhpaar nach einem der Ansprüche 1 bis 3, **dadurch gekennzeichnet, dass** die Greifhilfe (3, 3a, 5) jedes Handschuhs ein- oder mehrmals unterbrochen ist.

5. Handschuhpaar nach einem der vorhergehenden Ansprüche, **dadurch gekennzeichnet, dass** die Greifhilfe (3, 3a, 5) jedes Handschuhs mit die Griffigkeit erhöhenden Oberflächenstrukturen (8) versehen ist.

6. Handschuhpaar nach einem der vorhergehenden Ansprüche, **dadurch gekennzeichnet, dass** die Greifhilfe (3, 3a, 5) jedes Handschuhs einen von der Oberfläche der Stulpe (2) zumindest teilweise größer werdenden Querschnitt aufweist.

7. Handschuhpaar nach einem der vorhergehenden Ansprüche, **dadurch gekennzeichnet, dass** im Bereich der Greifhilfe (3, 3a, 5) jedes Handschuhs ein Dehnungsbereich (X) der Stulpe (2) vorgesehen ist.

8. Handschuhpaar nach einem der vorhergehenden Ansprüche, **dadurch gekennzeichnet, dass** die Greifhilfe (3, 3a, 5) jedes Handschuhs aus anderem, vorzugsweise griffigerem Material als die Stulpe (2) des Handschuhs angefertigt oder damit versehen ist.

9. Handschuhpaar nach einem der vorhergehenden Ansprüche, **dadurch gekennzeichnet, dass** die Greifhilfe (3, 3a, 5) und/oder zumindest der Übergangsbereich zwischen Stulpe (2) und Greifhilfe (3, 3a, 5) jedes Handschuhs verstärkt ist, beispielsweise mittels eingelagerter Verstärkungsfasern (9).

10. Verfahren zum Ausziehen eines Handschuhpaars nach einem der Ansprüche 1 bis 9, **dadurch gekennzeichnet, dass** mit der einen Hand des Trägers eine Greifhilfe (3, 5) des Handschuhs der anderen Hand ergriffen wird, welche an der Außenseite der Stulpe liegt, die den Handrückenteil der anderen Hand bedeckt, und mit der anderen Hand eine Greifhilfe (3, 5) des Handschuhs der einen Hand ergriffen wird, welche an einem die Handinnenseite der einen Hand des Trägers bedeckenden Umfangsabschnitt der Stulpe (2) des Handschuhs liegt, und in der Stellung der gegengleich parallel gehaltenen Arme und Hände des Trägers des Handschuhpaars jeweils eine Greifhilfe (3, 5) jedes Handschuhs von der jeweils anderen Hand ergriffen wird, und die Handschuhe gleichzeitig und gegengleich ausgezogen und während des Ausziehvorgang automatisch gewendet werden, ohne dass die verschmutzte oder kontaminierte Außenseite der Handschuhe mit der Haut des Trägers der Handschuhe in Kontakt kommt, und dass die Handschuhe des Handschuhpaars schließlich ganz gewendet und durch das weitere Ineinanderziehen miteinander verbunden werden.

## Claims

1. Pair of gloves, consisting of two gloves, in particular work gloves, examination gloves or surgical gloves, each having a uniform glove body made of fluid-tight, resilient material, for example rubber, the glove body comprising an actual hand part (1) and an adjoining cuff (2), a gripping aid (3, 3a, 5) being provided on each glove of the pair of gloves on the outer surface of the cuff (2) and so as to be spaced apart from the rear edge (4) of the cuff (2) in a peripheral portion, the gripping aid (3, 3a) of each glove being formed on the outside as a hollow protrusion of the glove that is open towards the inside of the glove, and the gripping aid (3, 3a, 5) of one glove of the pair of gloves and the gripping aid (3, 3a, 5) of the other glove of the pair of gloves being provided on adjacent peripheral portions of the cuff (2) and having an angular spacing of at least 135°, the gripping aid of one glove being arranged on the palm side, **characterised in that** the gripping aid of the other glove of the pair of gloves is arranged so as to be on the side on the back of the hand, and therefore, when the arms and hands are held in the parallel and mirror-inverted position, the gripping aids of each glove are gripped by the other hand in each case and simultaneously removed in a mirror-inverted manner and simultaneously automatically flipped, without the soiled or contaminated outside of the gloves coming into contact with the skin of the wearer, and **in that** the gloves of the pair of gloves are joined together by pulling said gloves further into one another.

2. Pair of gloves according to claim 1, **characterised in that** the gripping aids (3, 3a, 5) are arranged on both gloves so as to be substantially diametrically opposite.

3. Pair of gloves according to either claim 1 or claim 2, **characterised in that** the gripping aid (3, 3a, 5) of each glove extends over an angular range of at least 90°.

4. Pair of gloves according to any of claims 1 to 3, **characterised in that** the gripping aid (3, 3a, 5) of each glove is interrupted once or several times.

5. Pair of gloves according to any of the preceding claims, **characterised in that** the gripping aid (3, 3a, 5) of each glove is provided with surface structures (8) that increase the grip.

6. Pair of gloves according to any of the preceding claims, **characterised in that** the gripping aid (3, 3a, 5) of each glove has a cross section that becomes larger, at least in part, from the surface of the cuff (2).

7. Pair of gloves according to any of the preceding claims, **characterised in that** an expansion region (X) of the cuff (2) is provided in the region of the gripping aid (3, 3a, 5) of each glove.

8. Pair of gloves according to any of the preceding claims, **characterised in that** the gripping aid (3, 3a, 5) of each glove is made of or provided with material which is different from and preferably has more grip than the cuff (2) of the glove.

9. Pair of gloves according to any of the preceding claims, **characterised in that** the gripping aid (3, 3a, 5) and/or at least the transition region between the cuff (2) and gripping aid (3, 3a, 5) of each glove is reinforced, for example by means of embedded reinforcing fibres (9).

10. Method for removing a pair of gloves according to any of claims 1 to 9, **characterised in that** one hand of the wearer grips a gripping aid (3, 5) of the glove of the other hand, which gripping aid is on the outside of the cuff that covers the part on the back of the other hand, and the other hand grips a gripping aid (3, 5) of the glove of said one hand, which gripping aid is on a peripheral portion of the cuff (2) of the glove that covers the palm of said one hand of the wearer, and when the arms and hands of the wearer of the pair of gloves are held in the parallel and mirror-inverted position, a gripping aid (3, 5) of each glove is gripped by the other hand in each case, and the gloves are simultaneously removed in a mirror-inverted manner and are automatically flipped during the removal process, without the soiled or contaminated outside of the gloves coming into contact with the skin of the wearer of the gloves, and **in that** the gloves of the pair of gloves are finally flipped in their entirety and are joined together by pulling said gloves further into one another.

## Revendications

1. Paire de gants, constituée de deux gants, en particulier des gants de travail, médicaux ou chirurgicaux, comportant chacun un corps de gant uniforme, de préférence dans un matériau élastique, étanche aux liquides, tel que le caoutchouc, le corps de gant comportant une partie formant la main (1) proprement dite et une manchette (2) prolongeant cette dernière, au moins un moyen d'agrippement (3, 3a, 5) étant prévu sur chaque gant de la paire de gants sur la surface extérieure de la manchette (2) et à distance du bord arrière (4) de la manchette (2) sur une partie périphérique, le moyen d'agrippement (3, 3a) de chaque gant étant conçu comme une protubérance creuse du gant, ouverte vers l'intérieur du gant, réalisée vers l'extérieur et le moyen d'agrippement (3, 3a, 5) d'un gant de la paire de gants et le moyen d'agrippement (3, 3a, 5) de l'autre gant de la paire de gants étant prévues sur des portions périphériques adjacentes de la manchette (2) et présentant une distance angulaire d'au moins 135°, le moyen d'agrippement d'un gant étant disposé sur le côté intérieur de la main, **caractérisé en ce que** le moyen d'agrippement de l'autre gant de la paire de gant est disposé sur le dos de la main, de façon à ce que, lorsque les bras et les mains sont maintenus dans une position parallèle latéralement inversée, le moyen d'agrippement de chaque gant puisse être saisi par l'autre main et les gants puissent être retiré simultanément et de manière latéralement inversée et retournés simultanément automatiquement sans que la face extérieure sale ou contaminée du gant vienne en contact avec la peau de l'utilisateur, et à ce que les gants de la paire de gants puissent être reliés entre eux en continuant à les tirer l'un dans l'autre.

2. Paire de gants selon la revendication 1, **caractérisée en ce que** les moyens d'agrippement (3, 3a, 5) des deux gants sont disposés de manière sensiblement diamétralement opposée.

3. Paire de gants selon la revendication 1 ou 2, **caractérisée en ce que** le moyen d'agrippement (3, 3a, 5) de chaque gant s'étend sur une zone angulaire d'au moins 90°.

4. Paire de gants selon l'une quelconque des revendications 1 à 3, **caractérisée en ce que** le moyen d'agrippement (3, 3a, 5) de chaque gant est interrompu une ou plusieurs fois.

5. Paire de gants selon l'une quelconque des revendications précédentes, **caractérisée en ce que** le moyen d'agrippement (3, 3a, 5) de chaque gant est muni de structures de surface augmentant la capacité de préhension (8).

6. Paire de gants selon l'une des revendications précédentes, **caractérisée en ce que** le moyen d'agrippement (3, 3a, 5) de chaque gant présente une section transversale devant au moins partiellement plus grande à partir de la surface de la manchette (2).

7. Paire de gants selon l'une des revendications précédentes, **caractérisée en ce que**, au niveau du moyen d'agrippement (3, 3a, 5) de chaque gant est prévue une zone d'extension (X) de la manchette (2).

8. Paire de gants selon l'une quelconque des revendications précédentes, **caractérisée en ce que** le moyen d'agrippement (3, 3a, 5) de chaque gant est constitué d'un matériau différent, de préférence plus adhérent que la manchette (2) du gant ou est muni de celui-ci.

9. Paire de gants selon l'une des revendications précédentes, **caractérisée en ce que** le moyen d'agrippement (3, 3a, 5) et/ou au moins la zone de transition entre la manchette (2) et le moyen d'agrippement (3, 3a, 5) de chaque gant est renforcée, par exemple à l'aide de fibres de renforcement (9) intégrées.

10. Procédé permettant de retirer une paire de gants selon l'une des revendications 1 à 9, **caractérisé en ce qu'**une main de l'utilisateur saisit un moyen d'agrippement (3, 5) du gant de l'autre main, lequel est situé sur la face extérieure de la manchette, laquelle recouvre le dos de l'autre main et, avec l'autre main, un moyen d'agrippement (3, 5) du gant d'une main est saisi, qui se trouve au niveau d'une portion périphérique de la manchette (2) du gant recouvrant une main de l'utilisateur et, lorsque l'utilisateur de la paire de gants maintient ses bras et ses mains dans une position parallèle latéralement inversée, un moyen d'agrippement (3, 5) de chaque gant est saisi par l'autre main et les gants seront retirés simultanément et de manière latéralement inversée et seront retournés simultanément automatiquement pendant le processus de retrait des gants, sans que la face extérieure sale ou contaminée du gant vienne en contact avec la peau de l'utilisateur des gants et **en ce que** les gants de la paire de gants sont finalement complètement retournés et sont reliés entre eux par le fait que l'utilisateur continue à les tirer l'un dans l'autre.
